# EUROPÄISCHE PATENTSCHRIFT

(11) **EP 2 910 215 B1**
(45) Veröffentlichungstag und Bekanntmachung des Hinweises auf die Patenterteilung: **07.11.2018**
(21) Anmeldenummer: 14155880.9
(22) Anmeldetag: 20.02.2014
(51) Int. Cl.: A61C 1/02, A61C 1/14, A61C 1/18

(54) **Medizinisches, insbesondere dentales Handinstrument**
Medical, in particular dental hand instrument
Instrument manuel médical, en particulier dentaire

(43) Veröffentlichungstag der Anmeldung: 26.08.2015
(73) Patentinhaber: W & H Dentalwerk Bürmoos GmbH, 5111 Bürmoos (AT)
(72) Erfinder: WAGNER, Hannes, 5023 Salzburg (AT); EDER, Karlheinz, 5152 Michaelbeuern (AT); ROTHENWÄNDER, Michael, 5114 Göming (AT); REHRL, Hermann, 5112 Lamprechtshausen (AT)
(74) Vertreter: Benda, Ralf

(56) Entgegenhaltungen:
- EP-A2- 1 078 606
- EP-A2- 1 184 002
- WO-A1-2015/089239
- WO-A2-2013/076106

## Beschreibung

Die vorliegende Erfindung betrifft ein medizinisches, insbesondere dentales Handinstrument dessen Werkzeughaltevorrichtung in eine Hubbewegung versetzbar ist.

Aus der Patentanmeldung WO 2013/076106 A2 ist ein medizinisches, insbesondere dentales Handinstrument bekannt, dessen Werkzeughaltevorrichtung in eine simultane Hub- und Drehbewegung versetzbar ist. Zur Übertragung der Drehbewegung auf die Werkzeughaltevorrichtung ist eine Hohlwelle vorgesehen, an deren einem Ende ein Zahnrad angeordnet ist, das in ein weiteres Zahnrad an der Werkzeughaltevorrichtung eingreift. Zur Erzeugung der Hubbewegung ist ein Exzentergetriebe mit einem Exzenterstift und einem den Exzenterstift aufnehmenden Rücksprung an der Werkzeughaltevorrichtung vorgesehen. Der Exzenterstift ist an einer zweiten Wellen angeordnet, die in der Hohlwelle mit dem Zahnrad gelagert ist. Zur Reduktion der Drehzahl der Hohlwelle und damit der Werkzeughaltevorrichtung ist des Weiteren eine Untersetzungsgetriebe vorgesehen, so dass die Drehzahl der Werkzeughaltevorrichtung in Bezug auf die Hubfrequenz reduziert ist.

Die Patentanmeldung EP 1 078 606 A2 offenbart ein endodontisches Handstück mit einem Rotationsgetriebe und einem Hubgetriebe, wobei das antriebsseitige Element des Rotationsgetriebes und das antriebsseitige Element des Hubgetriebes an einer einzigen, gemeinsamen Antriebswelle derart angeordnet sind, dass eine Werkzeughaltevorrichtung des Handstücks in eine kontinuierliche Hubbewegung und eine diskontinuierliche Drehbewegung versetzbar ist.

Aus dem Stand der Technik gemäß Artikel 54(3) EPÜ ist die Patentanmeldung WO 2015/089239 A1 bekannt, die verschiedene Ausführungsbeispiele von Motorhandstücken mit Exzenter- und Zahnradgetrieben, die ein Werkzeug in eine gleichzeitige Hub- und Drehbewegung versetzen, offenbart.

Der vorliegenden Erfindung liegt die Aufgabe zugrunde ein medizinisches, insbesondere dentales, Handinstrument mit einer in eine simultane Hub- und Drehbewegung versetzbaren Werkzeughaltevorrichtung und einem im Vergleich zum Stand der Technik vereinfachten Getriebe zum simultanen Hub- und Drehantrieb der Werkzeughaltevorrichtung schaffen. Der vorliegenden Erfindung liegt auch die Aufgabe zugrunde ein medizinisches, insbesondere dentales, Handinstrument mit einem alternativen Hubgetriebe zu schaffen.

Diese Aufgabe wird gemäß der vorliegenden Erfindung durch ein medizinisches, insbesondere dentales, Handinstrument mit den Merkmalen des Anspruchs 1 gelöst. Besonders vorteilhafte Ausführungsformen sind in den Unteransprüchen angeführt.

Das medizinische, insbesondere dentale, Handinstrument umfasst: Eine Werkzeughaltevorrichtung zur, vorzugsweise lösbaren, Befestigung eines Behandlungswerkzeugs an dem Handinstrument, wobei die Werkzeughaltevorrichtung in eine simultane Hub- und Drehbewegung versetzbar ist, ein Rotationsgetriebe, welches ausgebildet ist, die Werkzeughaltevorrichtung in die Drehbewegung zu versetzten, und ein Hubgetriebe, welches ausgebildet ist, die Werkzeughaltevorrichtung in die Hubbewegung zu versetzten. Das Rotationsgetriebe weist zumindest ein antriebsseitiges Element und ein an der Werkzeughaltevorrichtung vorgesehenes abtriebsseitiges Element und das Hubgetriebe zumindest ein antriebsseitiges Element und ein an der Werkzeughaltevorrichtung vorgesehenes abtriebsseitiges Element auf. Das antriebsseitige Element des Rotationsgetriebes und das antriebsseitige Element des Hubgetriebes sind an einer einzigen, gemeinsamen Antriebswelle angeordnet. Damit ist der Aufbau des Handinstruments bzw. des Getriebes bei gleicher Funktion, i.e. simultanem Hub- und Drehantrieb der Werkzeughaltevorrichtung, erheblich vereinfacht. Weitere Vorteile sind eine einfachere und kostengünstigere Herstellung und Montage des Handinstruments und ein geringerer Platzverbrauch im Inneren des Handinstruments.

Die Werkzeughaltevorrichtung und die beiden Getriebe sind derart angeordnet oder ausgebildet, dass die Werkzeughaltevorrichtung in eine simultane Hub- und Drehbewegung versetzbar ist. Unter dem Begriff simultane Hub- und Drehbewegung wird eine gleichzeitigte rotierende Bewegung der Werkzeughaltevorrichtung um eine Dreh- oder Mittelachse der Werkzeughaltevorrichtung und Hubbewegung der Werkzeughaltevorrichtung entlang ihrer Dreh- oder Mittelachse verstanden. Das Hub- und das Rotationsgetriebe sind derart ausgebildet, dass die Werkzeughaltevorrichtung in eine kontinuierliche, simultane Hub- und Drehbewegung versetzbar ist. Unter einer kontinuierlichen, simultanen Hub- und Drehbewegung wird eine gleichzeitigte rotierende Bewegung der Werkzeughaltevorrichtung um eine Dreh- oder Mittelachse der Werkzeughaltevorrichtung und Hubbewegung der Werkzeughaltevorrichtung entlang ihrer Dreh- oder Mittelachse ohne (zumindest wesentlich) Unterbrechung der Hub- oder Drehbewegung verstanden. Die Werkzeughaltevorrichtung ist somit durch die Getriebe in eine beständige und simultane Hub- und Drehbewegung versetzbar.

Das Hub- und das Rotationsgetriebe sind an der Schnittstelle zwischen der einzigen, gemeinsamen Antriebswelle und der Werkzeughaltevorrichtung oder einem die Werkzeughaltevorrichtung aufweisenden Triebwellenteil angeordnet. Das Hub- und das Rotationsgetriebe sind vorzugsweise als separate oder getrennte Getriebe ausgebildet, so dass sie die Hubbewegung und Drehbewegung getrennt voneinander, insbesondere mittels unterschiedlicher Bauteile (im Folgenden zum Beispiel als erstes bis viertes Element bezeichnet), erzeugen und / oder übertragen. Das Hub- und das Rotationsgetriebe sind insbesondere in dem Verbindungsbereich zwischen dem Kopfabschnitt und dem Griffteil des Handinstruments angeordnet.

Das antriebsseitige Element des Rotationsgetriebes umfasst ein erstes Element und das antriebsseitige Element des Hubgetriebes umfasst ein zweites, separates und / oder von dem ersten Element unterschiedliches Element. Das erste oder antriebsseitige Element des Rotationsgetriebes weist ein Zahnrad auf. Das zweite oder antriebsseitige Element des Hubgetriebes weist zum Beispiel einen Stift, insbesondere einen länglichen und / oder zylindrischen Stift, auf. Vorzugsweise sind das antriebsseitige Element des Rotationsgetriebes und das antriebsseitige Element des Hubgetriebes an einer gemeinsamen Basis oder an einem gemeinsamen (End-)Abschnitt der einzigen, gemeinsamen Antriebswelle angeordnet oder befestigt.

Vorzugsweise sind zumindest ein Teil des antriebsseitigen Elements des Rotationsgetriebes und zumindest ein Teil des antriebsseitigen Elements des Hubgetriebes voneinander beabstandet. Bevorzugt sind das antriebsseitige Element des Rotationsgetriebes und das antriebsseitige Element des Hubgetriebes derart voneinander beabstandet, dass jeweils nur das antriebsseitige Element des Rotationsgetriebes mit dem abtriebsseitigen Element des Rotationsgetriebes und das antriebsseitige Element des Hubgetriebes und mit dem abtriebsseitigen Element des Hubgetriebes in eine Bewegung erzeugende und / oder übertragende Verbindung tritt.

Vorzugsweise hat das antriebsseitige Element des Rotationsgetriebes ein freies Ende und weist das antriebsseitige Element des Hubgetriebes ein freies Ende auf, wobei das freie Ende des antriebsseitigen Elements des Hubgetriebes zumindest geringfügig über das freie Ende des antriebsseitigen Elements des Rotationsgetriebes ragt.

Das an der Werkzeughaltevorrichtung vorgesehene, abtriebsseitige Element des Rotationsgetriebes umfasst ein drittes Element und das an der Werkzeughaltevorrichtung vorgesehene, abtriebsseitige Element des Hubgetriebes umfasst ein viertes, separates und / oder von dem dritten Element unterschiedliches Element. Das dritte oder abtriebsseitige Element des Rotationsgetriebes weist zum Beispiel ein Zahnrad auf. Vorzugsweise ist das abtriebsseitige Element des Rotationsgetriebes fest mit der Werkzeughaltevorrichtung verbunden, so dass das abtriebsseitige Element des Rotationsgetriebes mit der Werkzeughaltevorrichtung drehbar und entlang der Dreh- oder Mittelachse der Werkzeughaltevorrichtung verschiebbar ist.

Das vierte oder abtriebsseitige Element des Hubgetriebes weist zum Beispiel einen Rücksprung oder eine Nut auf, insbesondere einen kreisförmig geschlossenen und / oder ringförmigen Rücksprung. Der Rücksprung oder die Nut sind insbesondere in oder an der Werkzeughaltevorrichtung, insbesondere in oder an deren Außenfläche oder Außenmantel, oder in oder an einer die Werkzeughaltevorrichtung umgebenden Hülse, insbesondere in oder an deren Außenfläche oder Außenmantel, vorgesehen.

Das an der Werkzeughaltevorrichtung vorgesehene, abtriebsseitige Element des Rotationsgetriebes und das an der Werkzeughaltevorrichtung vorgesehene, abtriebsseitige Element des Hubgetriebes sind vorzugsweise voneinander beabstandet oder voneinander getrennt, zum Beispiel durch eine Schulter oder eine Stufe. Bevorzugt sind das abtriebsseitige Element des Rotationsgetriebes und das abtriebsseitige Element des Hubgetriebes derart voneinander beabstandet, dass jeweils nur das antriebsseitige Element des Rotationsgetriebes mit dem abtriebsseitigen Element des Rotationsgetriebes und das antriebsseitige Element des Hubgetriebes und mit dem abtriebsseitigen Element des Hubgetriebes in eine Bewegung erzeugende und / oder übertragende Verbindung tritt.

Das Zahnrad, welches Teil des ersten oder antriebsseitigen Elements des Rotationsgetriebes ist, ist konzentrisch um eine Drehachse der einzigen, gemeinsamen Antriebswelle angeordnet. Das Zahnrad ist somit durch die einzige, gemeinsame Antriebswelle ausschließlich in eine Drehbewegung versetzbar und ist insbesondere in Bezug auf die Mittelachse der Werkzeughaltevorrichtung translatorisch stationär, i.e. das Zahnrad ist in Bezug auf die Mittelachse der Werkzeughaltevorrichtung durch die einzige, gemeinsame Antriebswelle nicht in eine Längs- oder Hubbewegung versetzbar. Wenn, wie im Vorstehenden schon beschrieben, das abtriebsseitige Element des Rotationsgetriebes mit der Werkzeughaltevorrichtung entlang der Dreh- oder Mittelachse der Werkzeughaltevorrichtung verschiebbar ist, das antriebsseitige Element des Rotationsgetriebes jedoch nicht, so ist gemäß einer besonders bevorzugten Ausführungsform das abtriebsseitige Element des Rotationsgetriebes relativ zu dem antriebsseitige Element des Rotationsgetriebes entlang der Dreh- oder Mittelachse der Werkzeughaltevorrichtung verschiebbar.

Das Zahnrad, welches Teil des ersten oder antriebsseitigen Elements des Rotationsgetriebes ist, weist eine Hülse auf, in der zumindest ein Abschnitt der einzigen, gemeinsamen Antriebswelle aufgenommen ist. Bevorzugt schließt die Hülse, insbesondere einteilig, an den Zahnkranz des Zahnrads an. Bevorzugt ist die Hülse anschließend an eine Lagerhülse zur Lagerung der einzigen, gemeinsamen Antriebswelle angeordnet.

Vorzugsweise ist zumindest ein Teil des antriebsseitigen Elements, insbesondere der Stift, des Hubgetriebes innerhalb des Zahnrads, welches Teil des ersten oder antriebsseitigen Elements des Rotationsgetriebes ist, angeordnet. Insbesondere erstreckt sich zumindest ein Teil des antriebsseitigen Elements des Hubgetriebes durch das Zahnrad oder in das Innere des Zahnrads. Damit umgeben die Zähne des Zahnrads das antriebsseitige Element des Hubgetriebes, insbesondere umgeben die Zähne das antriebsseitige Element kreisförmig. Damit ist in vorteilhafter Weise ein besonders kompakter Aufbau des Handinstruments erreichbar.

Gemäß einem bevorzugten Ausführungsbeispiel ist das antriebsseitige Element, insbesondere der Stift, des Hubgetriebes konzentrisch um eine Drehachse der Antriebswelle, an welcher das antriebsseitige Element vorgesehen ist und durch welches es in Bewegung versetzbar ist, angeordnet. Besonders bevorzugt ist die Antriebswelle als einzige, gemeinsame Antriebswelle ausgebildet, an welcher in Übereinstimmung mit dem Vorstehenden zusätzlich das antriebsseitige Element des Rotationsgetriebes, insbesondere ein Zahnrad, angeordnet ist. Insbesondere weisen die Antriebswelle und das antriebsseitige Element des Hubgetriebes eine gemeinsame Drehachse auf.

Bevorzugt ist das konzentrisch um eine Drehachse der Antriebswelle angeordnete, antriebsseitige Element des Hubgetriebes einteilig mit der Antriebswelle oder mit der einzigen, gemeinsamen Antriebswelle ausgebildet. Insbesondere ist das konzentrisch angeordnete Element als, insbesondere stiftförmiger, Fortsatz oder Stummel an der (insbesondere einzigen, gemeinsamen) Antriebswelle ausgebildet. Insbesondere ist das konzentrisch angeordnete Element an einem Ende oder an einer Endfläche der (insbesondere einzigen, gemeinsamen) Antriebswelle angeordnet.

Bevorzugt ist das konzentrisch um eine Drehachse der Antriebswelle angeordnete, antriebsseitige Element des Hubgetriebes derart ausgebildet, dass bei einer vollständigen Drehung des antriebsseitigen Elements des Hubgetriebes, d.h. bei einer Drehung um 360°, die Werkzeughaltevorrichtung mehr als einen Doppelhub, insbesondere zumindest zwei Doppelhübe, durchläuft. In anderen Worten ist die Werkzeughaltevorrichtung bei einer vollständigen Drehung des konzentrisch angeordneten, antriebsseitigen Elements des Hubgetriebes von einer Ausgangsposition über einen Aufwärts- oder Vorhub, einen Abwärts- oder Rückhub wieder in die Ausgangsposition und über diese Ausgangsposition hinaus bewegbar. Damit ist in vorteilhafter Weise eine Erhöhung der Hubfrequenz in Bezug auf die Drehzahl der Antriebswelle oder der Werkzeughaltevorrichtung erzielbar.

Bevorzugt weist das konzentrisch um eine Drehachse der Antriebswelle angeordnete, antriebsseitige Element des Hubgetriebes einen, insbesondere im Querschnitt, polygonalen Stift auf, der insbesondere in einen Rücksprung des abtriebsseitigen Elements des Hubgetriebes an der Werkzeughaltevorrichtung eingreift. Vorzugsweise ist der polygonale Stift einteilig mit der (insbesondere einzigen, gemeinsamen) Antriebswelle ausgebildet. Vorzugsweise umfasst der polygonale Stift eine ungerade Anzahl an Ecken oder Scheiteln, insbesondere drei oder fünf Scheitel. Besonders bevorzugt weist der polygonale Stift nach außen gewölbte oder konvexe Außenseiten auf. Besonders bevorzugt ist der polygonale Stift derart ausgebildet, dass (im Querschnitt) die Entfernung von einem Scheitel zu der dem Scheitel gegenüber liegenden Außenseite entlang der gesamten Außenseite konstant ist. Besonders bevorzugt ist der polygonale Stift als Gleichdick ausgebildet, zum Beispiel als drei- oder fünfeckiger Gleichdick.

Bevorzugt umfasst das abtriebsseitige Element oder der Rücksprung des Hubgetriebes an der Werkzeughaltevorrichtung, in welches/welchen der konzentrisch angeordnete, polygonale Stift eingreift, zwei einander gegenüberliegende Flächen, insbesondere Ringflächen. Besonders bevorzugt sind der polygonale Stift oder der Gleichdick und das abtriebsseitige Element derart bemessen und / oder angeordnet und / oder ausgebildet, dass der polygonale Stift oder der Gleichdick kontinuierlich die beiden Flächen des abtriebsseitigen Elements kontaktiert. Damit werden in vorteilhafter Weise eine hohe Laufruhe und eine geringe Lärmerzeugung erzielt.

Bevorzugt ist im Querschnitt der Radius der nach außen gewölbten Außenseiten des konzentrisch angeordneten, polygonalen Stifts größer als der Radius des zylindrischen Außenmantels der einzigen, gemeinsamen Antriebswelle. Besonders bevorzugt ist im Querschnitt und bezogen auf die Drehachse der einzigen, gemeinsamen Antriebswelle die Entfernung von der Drehachse zu den nach außen gewölbten Außenseiten des konzentrisch angeordneten, polygonalen Stifts geringer als der Radius des zylindrischen Außenmantels der einzigen, gemeinsamen Antriebswelle. Damit ist in vorteilhafter Weise ein besonders kompakter Aufbau der Antriebswelle und des antriebsseitigen Elements des Hubgetriebes erzielbar.

Gemäß einem alternativen Ausführungsbeispiel ist das antriebsseitige Element des Hubgetriebes exzentrisch zu eine Drehachse der einzigen, gemeinsamen Antriebswelle angeordnet. Bevorzugt weist das antriebsseitige Element des Hubgetriebes einen, vorzugsweise einteilig mit der einzigen, gemeinsamen Antriebswelle ausgebildeten, zylindrischen Stift auf, der insbesondere in das abtriebsseitige Element des Hubgetriebes, zum Beispiel einen Rücksprung, an der Werkzeughaltevorrichtung eingreift. Bevorzugt ist der zylindrische Stift von einer Buchse umgeben, die mit dem zylindrischen Stift bewegbar ist.

Die Erfindung wird nachfolgend anhand bevorzugter Ausführungsbeispiele und Bezug nehmend auf die beigefügten Zeichnungen erläutert. Es zeigt die
Figur 1 ein erstes Ausführungsbeispiel eines medizinischen, insbesondere dentalen, Handinstruments mit einer Werkzeughaltevorrichtung, die mittels einem Rotationsgetriebe und einem Hubgetriebe in eine simultane Hub- und Drehbewegung versetzbar ist, wobei das antriebsseitige Element des Rotationsgetriebes und das exzentrisch angeordnete, antriebsseitige Element des Hubgetriebes an einer einzigen, gemeinsamen Antriebswelle vorgesehen sind;
Figur 2 eine vergrößerte Darstellung des Kopfabschnitts der Handinstruments der Figur 1;
Figur 3 ein zweites Ausführungsbeispiel eines medizinischen, insbesondere dentalen, Handinstruments mit einer Werkzeughaltevorrichtung, die mittels einem Rotationsgetriebe und einem Hubgetriebe in eine simultane Hub- und Drehbewegung versetzbar ist, wobei das antriebsseitige Element des Rotationsgetriebes und das konzentrisch angeordnete, antriebsseitige Element des Hubgetriebes an einer einzigen, gemeinsamen Antriebswelle vorgesehen sind;
Figur 4A eine Vorderansicht einer Antriebswelle und eines daran befestigten konzentrisch angeordneten, antriebsseitigen Elements des Hubgetriebes;
Figur 4B eine Seitenansicht der Antriebswelle und des daran befestigten konzentrisch angeordneten, antriebsseitigen Elements des Hubgetriebes der Figur 4A.

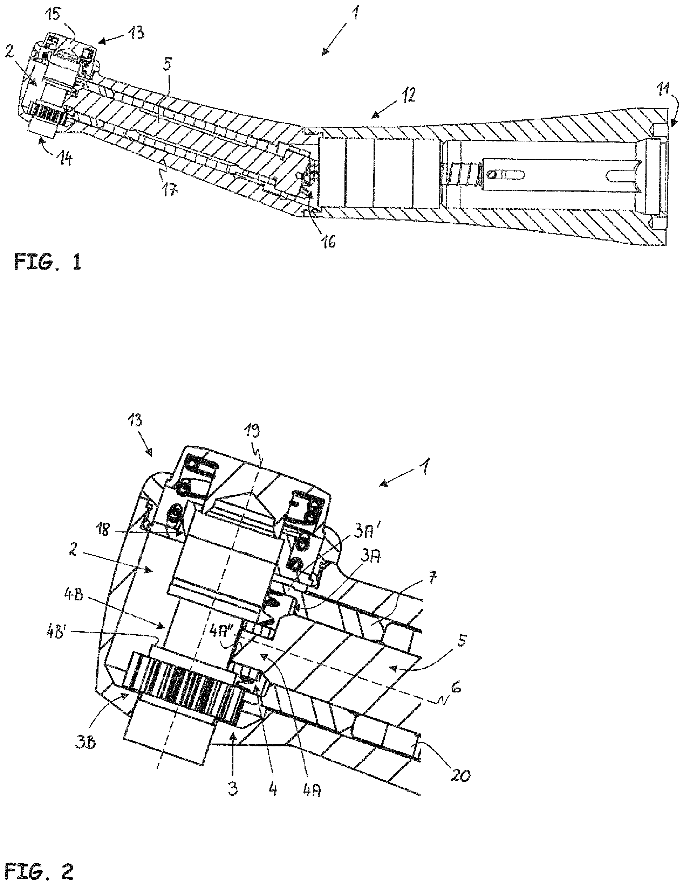

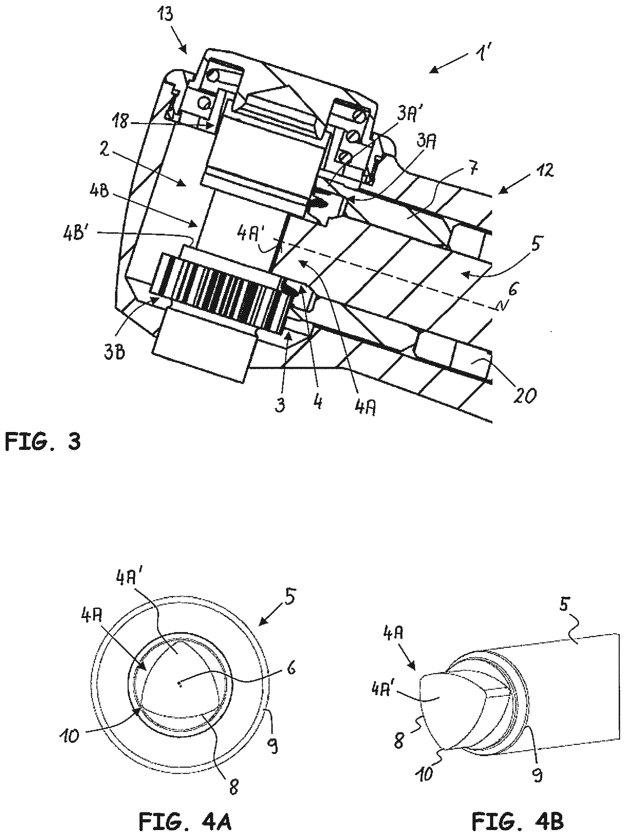

Das in den Figuren 1 und 3 dargestellte medizinische, insbesondere dentale, Handinstrument 1, 1' ist als längliches, rohrförmiges, mit einer Hand haltbares Instrument oder Handstück ausgebildet. Das Handinstrument 1, 1' umfasst eine Außenhülse 17. An einem Ende des Handinstruments 1, 1' ist eine Anschlussvorrichtung oder Kupplung 11 zum lösbaren Verbinden zum Beispiel an eine Steuervorrichtung, an ein Antriebseinheit, an eine Energiequelle und / oder an eine Fluidquelle, insbesondere eine Wasser- und / oder Druckluftquelle vorgesehen. Das Handinstrument 1, 1' umfasst des Weiteren einen gebogenen oder einen zwei gewinkelt zueinander angeordnete Abschnitte aufweisenden Griffteil 12 und einen daran anschließenden Kopfabschnitt 13.

In der Außenhülse des Kopfabschnitts 13 ist eine Werkzeugöffnung 14 vorgesehen, durch die ein Werkzeug zum Einwirken auf eine Behandlungsstelle lösbar in den

Kopfabschnitt 13 einbringbar ist. Im Kopfabschnitt 13 ist eine lösbare Werkzeughaltevorrichtung 2, die zum Beispiel eine Spannzange umfasst, angeordnet, welche das Werkzeug lösbar am Kopfabschnitt 13 befestigt. Die Werkzeugöffnung 14 ist seitlich am Kopfabschnitt 13 angeordnet, so dass das Werkzeug gewinkelt zum Griffteil 12 oder dessen Längsachse aus dem Kopfabschnitt 13 ragt. An dem der Werkzeugöffnung 14 gegenüberliegenden Ende des Kopfabschnitts 13 ist ein Drucktaster 15 vorgesehen, der mit einer im Kopfabschnitt 13 angeordneten Werkzeuglösevorrichtung zusammenwirkt, um das Werkzeug aus dem Kopfabschnitt 13 oder aus der Werkzeughaltevorrichtung 2 frei zu geben. Selbstverständlich kann das Handinstrument 1, 1' auch andere bekannte Formen aufweisen, zum Beispiel pistolenförmig oder gerade ausgebildet sein.

Von der Anschlussvorrichtung 11 durch den Griffteil 12 erstreckt sich eine Vorrichtung zur Übertragung von Antriebsenergie auf die Werkzeughaltevorrichtung 2. Die Vorrichtung zur Übertragung von Antriebsenergie umfasst zum Beispiel zumindest eine Antriebswelle 5 und / oder zumindest ein Getriebe 16. Die Übertragung der Antriebsbewegung von der anschließend an den Kopfabschnitt 13 angeordneten Antriebswelle 5 auf die Werkzeughaltevorrichtung 2 mittels einer ein Rotationsgetriebe 3 und ein Hubgetriebe 4 aufweisenden Getriebeeinheit, die im Folgenden noch detaillierter beschrieben wird.

Von der Anschlussvorrichtung 11 durch den Griffteil 12 erstreckt / erstrecken sich bevorzugt auch zumindest eine Fluid- oder Medienleitung, zum Beispiel für Wasser oder Druckluft, und / oder ein Lichtleiter und / oder eine elektrische Versorgungs- oder Steuerleitungen.

Im Folgenden wird unter Bezugnahme auf die Figuren 2 und 3 der Aufbau der Kopfabschnitte 13 des Handinstruments 1 und des Handinstruments 1' beschrieben:
Die Werkzeughaltevorrichtung 2 zur Befestigung eines Behandlungswerkzeugs an dem Handinstrument 1, 1' ist in eine simultane Hub- und Drehbewegung versetzbar. Die Werkzeughaltevorrichtung 2 umfasst insbesondere eine Dreh- oder Mittelachse 19, wobei sie um die Mittelachse 19 drehbar und entlang der Mittelachse 19 verschiebbar, insbesondere wechselweise hin und her verschiebbar, ausgebildet ist. Dazu ist im Kopfabschnitt 13 zumindest ein Lager 18, insbesondere ein Gleitlager, vorgesehen, das die Werkzeughaltevorrichtung 2 derart lagert, dass die Werkzeughaltevorrichtung 2 in die simultane Hub- und Drehbewegung versetzbar ist.

Zum Versetzen der Werkzeughaltevorrichtung 2 in die Drehbewegung ist ein Rotationsgetriebe 3 vorgesehen. Ein Hubgetriebe 4 ist ausgebildet, die Werkzeughaltevorrichtung 2 in die Hubbewegung zu versetzten. Die beiden Getriebe 3, 4 sind unabhängig oder separat voneinander zur Übertragung der Drehbewegung bzw. Erzeugung der Hubbewegung ausgebildet.

Das Rotationsgetriebe 3 weist zumindest ein antriebsseitiges Element 3A, zum Beispiel ein Zahnrad 3A', und ein an der Werkzeughaltevorrichtung 2 vorgesehenes abtriebsseitiges Element 3B auf. Das Zahnrad 3A' ist konzentrisch um eine Drehachse 6 der Antriebswelle 5 angeordnet. Das abtriebsseitige Element 3B umfasst insbesondere auch ein Zahnrad. Das abtriebsseitige Element 3B ist insbesondere zwischen der Werkzeugöffnung 14 und einem antriebsseitigen Element 4A oder einem abtriebsseitigen Element 4B des Hubgetriebes 4 angeordnet. Die beiden Element 3A, 3B sind derart ausgebildet und/ oder angeordnet, dass sie operativ miteinander verbunden sind, insbesondere miteinander kämmen, so dass eine Drehbewegung von dem antriebsseitigen Element 3A auf das abtriebsseitige Element 3B übertragbar ist.

Das Hubgetriebe 4 umfasst zumindest ein antriebsseitiges Element 4A und ein an der Werkzeughaltevorrichtung 2 vorgesehenes abtriebsseitiges Element 4B. Das antriebsseitige Element 4A weist einen Stift 4A', 4A" auf. Das abtriebsseitige Element 4B des Hubgetriebes 4 ist an der Werkzeughaltevorrichtung 2 oder einer die Werkzeughaltevorrichtung 2 umgebenden Hülse vorgesehen und umfasst insbesondere einen Rücksprung 4B' oder eine ringförmige Nut. Das abtriebsseitige Element 4B des Hubgetriebes 4 ist insbesondere zwischen dem abtriebsseitigen Element 3B des Rotationsgetriebes 3 und dem Drucktaster 15 angeordet.

Die Antriebswelle 5 ist zur Übertragung einer Drehbewegung ausgebildet oder um die Drehachse 6 in eine Drehbewegung versetzbar. Die Antriebswelle ist als einzige, gemeinsame Antriebswelle 5 für das antriebsseitige Element 3A des Rotationsgetriebes 3 und für das antriebsseitige Element 4A des Hubgetriebes 4 ausgebildet, so dass das Rotationsgetriebe 3 und das Hubgetriebe 4 gemeinsam und ausschließlich durch diese eine gemeinsame Antriebswelle 5 antreibbar sind.

Das antriebsseitige Element 4A des Hubgetriebes 4, insbesondere die Stifte 4A', 4A", sind vorzugsweise einteilig mit der einzigen, gemeinsamen Antriebswelle 5 verbunden. Zum Verbinden der einzigen, gemeinsamen Antriebswelle 5 mit dem antriebsseitigen Element 3A des Rotationsgetriebes 3, insbesondere dem Zahnrads 3A', weist das antriebsseitige Element 3A eine Hülse 7 auf, in der zumindest ein Abschnitt der einzigen, gemeinsamen Antriebswelle 5 aufgenommen ist, zum Beispiel durch Verpressen. An die Hülse 7 schließt ein in Bezug auf Antriebswelle 5 stationäre Lagerhülse 20 zur Lagerung der einzigen, gemeinsamen Antriebswelle 5 an.

Zumindest ein Teil des antriebsseitigen Elements 4A des Hubgetriebes 4, insbesondere zumindest ein Teil des Stift 4A', 4A", erstreckt sich durch das Zahnrad 3A' oder in das Innere der ringförmig angeordneten Zahnreihe des Zahnrads 3A', so dass die Zähne des Zahnrads 3A' das antriebsseitige Element 4A des Hubgetriebes 4 umgeben.

Wie im Folgenden dargestellt, unterscheiden sich die Handinstrumente 1, 1' der Figuren 2 und 3 insbesondere in der Ausgestaltung und Anordnung der antriebsseitigen Elemente 4A der jeweiligen Hubgetriebe 4.

Das antriebsseitige Element 4A des Hubgetriebes 4 der Figur 2 ist exzentrisch zu der Drehachse 6 der einzigen, gemeinsamen Antriebswelle 5 und / oder zu dem antriebsseitigen Element 3A des Rotationsgetriebes 3, insbesondere zu der ringförmig angeordneten Zahnreihe des Zahnrads 3A', angeordnet. Das antriebsseitige Element 4A umfasst einen, vorzugsweise einteilig mit der einzigen, gemeinsamen Antriebswelle 5 ausgebildeten, zylindrischen Stift 4A". Der zylindrischen Stift 4A" ist derart bemessen und / oder an einem freien Ende der einzigen, gemeinsamen Antriebswelle 5 oder an einer Endfläche der Antriebswelle 5 angeordnet, dass er in das abtriebsseitige Element 4B des Hubgetriebes 4 an der Werkzeughaltevorrichtung 2 eingreift.

Das antriebsseitige Element 4A des Hubgetriebes 4 der Figur 3 ist konzentrisch um die Drehachse 6 der einzigen, gemeinsamen Antriebswelle 5 angeordnet. Das antriebsseitige Element 4A des Hubgetriebes 4 ist des Weiteren derart ausgebildet, dass bei einer vollständigen Drehung des antriebsseitigen Elements 4A des Hubgetriebes 4 die Werkzeughaltevorrichtung 2 mehr als einen Doppelhub durchläuft.

Das antriebsseitige Element 4A des Hubgetriebes 4 der Figur 3 umfasst einen, vorzugsweise einteilig mit der einzigen, gemeinsamen Antriebswelle 5 ausgebildeten, polygonalen Stift 4A', der in das abtriebsseitige Element 4B in Form eines ringförmigen Rücksprungs 4B' des Hubgetriebes 4 an der Werkzeughaltevorrichtung 2 eingreift.

Der an der einzigen, gemeinsamen Antriebswelle 5 vorgesehene polygonale Stift 4A' ist in den Figuren 4A, 4B im Detail dargestellt. Der polygonale Stift 4A' weist im Querschnitt eine dreieckige Form mit drei Ecken oder Scheiteln 10 auf. Die Scheiteln 10 werden durch nach außen gewölbte oder konvexe Außenseiten 8 miteinander verbunden.

Die Außenabmessung des polygonalen Stifts 4A' ist im Querschnitt geringer als die Außenabmessung der einzigen, gemeinsamen Antriebswelle 5. Insbesondere ist im Querschnitt der Radius der nach außen gewölbten Außenseiten 8 des polygonalen Stifts 4A' größer als der Radius des zylindrischen Außenmantels 9 der einzigen, gemeinsamen Antriebswelle 5.

Es sind alle Merkmale aller beschriebenen und dargestellten Ausführungsbeispiele miteinander kombinierbar.

## Patentansprüche

1. Medizinisches, insbesondere dentales, Handinstrument (1, 1'), umfassend: eine Werkzeughaltevorrichtung (2) zur Befestigung eines Behandlungswerkzeugs an dem Handinstrument (1, 1'), wobei die Werkzeughaltevorrichtung (2) in eine simultane Hub- und Drehbewegung versetzbar ist, ein Rotationsgetriebe (3), welches ausgebildet ist, die Werkzeughaltevorrichtung (2) in die Drehbewegung zu versetzten, und ein Hubgetriebe (4), welches ausgebildet ist, die Werkzeughaltevorrichtung (2) in die Hubbewegung zu versetzten, wobei das Rotationsgetriebe (3) zumindest ein antriebsseitiges Element (3A), welches ein Zahnrad (3A') aufweist, und ein an der Werkzeughaltevorrichtung (2) vorgesehenes abtriebsseitiges Element (3B) aufweist, und wobei das Hubgetriebe (4) zumindest ein antriebsseitiges Element (4A) und ein an der Werkzeughaltevorrichtung (2) vorgesehenes abtriebsseitiges Element (4B) aufweist, wobei das antriebsseitige Element (3A) des Rotationsgetriebes (3) und das antriebsseitige Element (4A) des Hubgetriebes (4) an einer einzigen, gemeinsamen Antriebswelle (5) angeordnet sind, wobei die Antriebswelle (5) um eine Drehachse (6) in eine Drehbewegung versetzbar ist und das Rotationsgetriebe (3) und das Hubgetriebe (4) gemeinsam und ausschließlich durch diese einzige, gemeinsame Antriebswelle (5) antreibbar sind, wobei das antriebsseitige Zahnrad (3A') konzentrisch um die Drehachse (6) der einzigen, gemeinsamen Antriebswelle (5) angeordnet ist, wobei
das Hubgetriebe (4) und das Rotationsgetriebe (3) zumindest gemäß einem der folgenden Merkmale(i) - (iii) ausgebildet sind, wodurch die Werkzeughaltevorrichtung (2) in eine kontinuierliche, simultane Hub- und Drehbewegung versetzbar ist:
(i) das abtriebsseitige Element (3B) des Rotationsgetriebes (3) ist fest mit der Werkzeughaltevorrichtung (2) verbunden, wodurch das abtriebsseitige Element (3B) des Rotationsgetriebes (3) mit der Werkzeughaltevorrichtung (2) drehbar und entlang einer Dreh- oder Mittelachse (19) der Werkzeughaltevorrichtung (2) verschiebbar ist;
(ii) das antriebsseitige Element (3A) des Rotationsgetriebes (3) und das antriebsseitige Element (4A) des Hubgetriebes (4) weisen jeweils ein freies Ende auf, wobei das freie Ende des antriebsseitigen Elements (4A) des Hubgetriebes (4) zumindest geringfügig über das freie Ende des antriebsseitigen Elements (3A) des Rotationsgetriebes (3) ragt;
(iii) das abtriebsseitige Element (3B) des Rotationsgetriebes (3) und das abtriebsseitige Element (4B) des Hubgetriebes (4) sind derart voneinander beabstandet, dass jeweils nur das antriebsseitige Element (3A) des Rotationsgetriebes (3) mit dem abtriebsseitigen Element (3B) des Rotationsgetriebes (3) und das antriebsseitige Element (4A) des Hubgetriebes (4) und mit dem abtriebsseitigen Element (4B) des Hubgetriebes (4) in eine Bewegung erzeugende und / oder übertragende Verbindung tritt, und wobei
das antriebsseitige Zahnrad (3A') eine sich in einen Griffabschnitt (12) des Handinstruments (1, 1') erstreckende Hülse (7) aufweist, in der zumindest ein Abschnitt der einzigen, gemeinsamen Antriebswelle (5) aufgenommen ist.

2. Medizinisches, insbesondere dentales, Handinstrument (1, 1') nach Anspruch 1, **dadurch gekennzeichnet, dass**
das abtriebsseitige Element (3B) des Rotationsgetriebes (3) ein Zahnrad aufweist.

3. Medizinisches, insbesondere dentales, Handinstrument (1, 1') nach Anspruch 1 oder 2, **dadurch gekennzeichnet, dass**
das Hubgetriebe (4) und das Rotationsgetriebe (3) als separate Getriebe ausgebildet sind, so dass sie die Hubbewegung und Drehbewegung getrennt voneinander, insbesondere mittels unterschiedlicher Bauteile, erzeugen und / oder übertragen.

4. Medizinisches, insbesondere dentales, Handinstrument (1, 1') nach Anspruch 1, 2 oder 3, **dadurch gekennzeichnet, dass**
der zumindest eine Abschnitt der einzigen, gemeinsamen Antriebswelle (5) durch Verpressen in der Hülse (7) aufgenommen ist.

5. Medizinisches, insbesondere dentales, Handinstrument (1, 1') nach einem der vorstehenden Ansprüche, **dadurch gekennzeichnet, dass**
sich zumindest ein Teil des antriebsseitigen Elements (4A) des Hubgetriebes (4) durch das antriebsseitige Zahnrad (3A') erstreckt, so dass die Zähne des antriebsseitige Zahnrads (3A') das antriebsseitige Element (4A) des Hubgetriebes (4) umgeben.

6. Medizinisches, insbesondere dentales, Handinstrument (1, 1') nach einem der vorstehenden Ansprüche, **dadurch gekennzeichnet, dass**
das antriebsseitige Element (4A) des Hubgetriebes (4) konzentrisch um eine Drehachse (6) der einzigen, gemeinsamen Antriebswelle (5) angeordnet ist.

7. Medizinisches, insbesondere dentales, Handinstrument (1, 1') nach Anspruch 6, **dadurch gekennzeichnet, dass**
das antriebsseitige Element (4A) des Hubgetriebes (4) einen, vorzugsweise einteilig mit der einzigen, gemeinsamen Antriebswelle (5) ausgebildeten, polygonalen Stift (4A') aufweist, der in das abtriebsseitige Element (4B) des Hubgetriebes (4) an der Werkzeughaltevorrichtung (2) eingreift.

8. Medizinisches, insbesondere dentales, Handinstrument (1, 1') nach Anspruch 7, **dadurch gekennzeichnet, dass**
der polygonale Stift (4A') als Gleichdick ausgebildet ist.

9. Medizinisches, insbesondere dentales, Handinstrument (1, 1') nach Anspruch 7 oder 8, **dadurch gekennzeichnet, dass**
der polygonale Stift (4A') nach außen gewölbte Außenseiten (8) aufweist.

10. Medizinisches, insbesondere dentales, Handinstrument (1, 1') nach Anspruch 9, **dadurch gekennzeichnet, dass**
im Querschnitt der Radius der nach außen gewölbten Außenseiten (8) des polygonalen Stifts (4A') größer ist als der Radius des zylindrischen Außenmantels (9) der einzigen, gemeinsamen Antriebswelle (5).

11. Medizinisches, insbesondere dentales, Handinstrument (1, 1') nach einem der Ansprüche 7 - 10, **dadurch gekennzeichnet, dass**
der polygonale Stift (4A') drei oder fünf Scheitel (10) aufweist.

12. Medizinisches, insbesondere dentales, Handinstrument (1, 1') nach einem der Ansprüche 1 - 5, **dadurch gekennzeichnet, dass**
das antriebsseitige Element (4A) des Hubgetriebes (4) exzentrisch zu einer Drehachse (6) der einzigen, gemeinsamen Antriebswelle (5) angeordnet ist.

13. Medizinisches, insbesondere dentales, Handinstrument (1, 1') nach Anspruch 12, **dadurch gekennzeichnet, dass**
das antriebsseitige Element (4A) des Hubgetriebes (4) einen zylindrischen Stift (4A") aufweist, der in das abtriebsseitige Element (4B) des Hubgetriebes (4) an der Werkzeughaltevorrichtung (2) eingreift.

14. Medizinisches, insbesondere dentales, Handinstrument (1, 1') nach einem der vorstehenden Ansprüche, **dadurch gekennzeichnet, dass**
das antriebsseitige Element (4A) des Hubgetriebes (4) einteilig mit der einzigen, gemeinsamen Antriebswelle (5) verbunden ist.

15. Medizinisches, insbesondere dentales, Handinstrument (1, 1') nach einem der vorstehenden Ansprüche, **dadurch gekennzeichnet, dass**
das abtriebsseitige Element (4B) des Hubgetriebes (4) einen Rücksprung aufweist, der kreisförmig geschlossen oder ringförmig ist.

## Claims

1. A medical, in particular dental, hand instrument (1, 1'), comprising: a tool-holding device (2) for fastening a treatment tool on the hand instrument (1, 1'), wherein the tool-holding device (2) can be induced to a simultaneous stroke and rotational movement, a rotary gear (3) which is designed to induce the tool-holding device (2) to a rotational movement, and a stroke gear (4) which is designed to induce the tool-holding device (2) to a stroke movement, wherein the rotary gear (3) has at least one drive-side element (3A) which comprises a gearwheel (3A') and one output-side element (3B) which is provided on the tool-holding device (2) and wherein the stroke gear (4) comprises at least one drive-side element (4A) and one output-side element (4B), which is provided on the tool-holding device (2), wherein the drive-side element (3A) of the rotary gear (3) and the drive-side element (4A) of the stroke gear (4) are disposed on a single, joint driveshaft (5), wherein the driveshaft (5) can be induced to a rotary movement about an axis of rotation (6) and the rotary gear (3) and the stroke gear (4) can be driven jointly and exclusively by this single, joint driveshaft (5), wherein the drive-side gearwheel (3A') is arranged concentrically about the axis of rotation (6) of the single, joint driveshaft (5), wherein
the rotary gear (3) and the stroke gear (4) are configured according to at least one of the following characteristics (i) - (iii) which induce(s) the tool-holding device (2) to a simultaneous stroke and rotational movement:
(i) the output-side element (3B) of the rotary gear (3) is coupled fixedly to the tool-holding device (2), so that the output-side element (3B) of the rotary gear (3) rotates with the tool-holding device (2) and is displaceable along the axis of rotation or the central axis (19) of the tool-holding device (2);
(ii) the drive-side element (3A) of the rotary gear (3) and the drive-side element (4A) of the stroke gear (4) each comprise a free end, wherein the free end of the drive-side element (4A) of the stroke gear (4) protrudes at least slightly beyond the free end of the drive-side element (3A) of the rotary gear (3);
(iii) the output-side element (3B) of the rotary gear (3) and the output-side element (4B) of the stroke gear (4) are spaced a distance apart from one another such, that only the drive-side element (3A) of the rotary gear (3) enters into a motion-generating and/or -transmitting connection with the output-side element (3B) of the rotary gear (3), and the drive-side element (4A) of the stroke gear (4) enters into a motion-generating and/or -transmitting connection with the output-side element (4B) of the stroke gear (4), and wherein
the drive-side gearwheel (3A') comprises a sleeve (7) which extends into a handle part (12) of the hand instrument (1, 1') and in which at least a section of the single, joint driveshaft (5) is accommodated.

2. The medical, in particular dental, hand instrument (1, 1') according to claim 1, **characterized in that**
the output-side element (3B) of the rotary gear (3) comprises a gearwheel.

3. The medical, in particular dental, hand instrument (1, 1') according to claim 1 or 2, **characterized in that**
the stroke gear (4) and rotary gear (3) are designed as separate gears, so that they generate and/or transmit the stroke movement and the rotational movement separately from one another, in particular through different components.

4. The medical, in particular dental, hand instrument (1, 1') according to claim 1, 2 or 3, **characterized in that**
the at least one section of the single, joint driveshaft (5) is accommodated in the sleeve (7) by press-fitting.

5. The medical, in particular dental, hand instrument (1, 1') according to any one of the preceding claims, **characterized in that**
at least a portion of the drive-side element (4A) of the stroke gear (4) extends through the drive-side gearwheel (3A'), so that the teeth of the drive-side gearwheel (3A') surround the drive-side element (4A) of the stroke gear (4).

6. The medical, in particular dental, hand instrument (1, 1') according to any one of the preceding claims, **characterized in that**
the drive-side element (4A) of the stroke gear (4) is disposed concentrically around an axis of rotation (6) of the single, joint driveshaft (5).

7. The medical, in particular dental, hand instrument (1, 1') according to claim 6, **characterized in that**
the drive-side element (4A) of the stroke gear (4) comprises a polygonal pin (4A'), which is preferably designed in one piece with the single, joint driveshaft (5) and engages the output-side element (4B) of the stroke gear (4) on the tool-holding device (2).

8. The medical, in particular dental, hand instrument (1, 1') according to claim 7, **characterized in that**
the polygonal pin (4A') is designed as an orbiform element.

9. The medical, in particular dental, hand instrument (1, 1') according to claim 7 or 8, **characterized in that**
the polygonal pin (4A') comprises outwardly bulging exterior sides (8).

10. The medical, in particular dental, hand instrument (1, 1') according to claim 9, **characterized in that**
in cross section the radius of the outwardly bulging exterior sides (8) of the polygonal pin (4A') is larger than the radius of the cylindrical outer circumference (9) of the single, joint driveshaft (5).

11. The medical, in particular dental, hand instrument (1, 1') according to any one of claims 7 - 10, **characterized in that**
the polygonal pin (4A') comprises three or five peaks (10).

12. The medical, in particular dental, hand instrument (1, 1') according to one of claims 1 - 5, **characterized in that**
the drive-side element (4A) of the stroke gear (4) is disposed eccentrically to an axis of rotation (6) of the single, joint driveshaft (5).

13. The medical, in particular dental, hand instrument (1, 1') according to claim 12, **characterized in that**
the drive-side element (4A) of the stroke gear (4) comprises a cylindrical pin (4A"), which engages the output-side element (4B) of the stroke gear (4) on the tool-holding device (2).

14. The medical, in particular dental, hand instrument (1, 1') according to any one of the preceding claims, **characterized in that**
the drive-side element (4A) of the stroke gear (4) is connected in one piece with the single, joint driveshaft (5).

15. The medical, in particular dental, hand instrument (1, 1') according to any one of the preceding claims, **characterized in that**
the output-side element (4B) of the stroke gear (4) comprises a setback which is closed in a circle or annular.

## Revendications

1. Instrument manuel médical, en particulier dentaire (1, 1'), comprenant: un dispositif porte-outil (2) pour la fixation d'un outil de traitement sur l'instrument manuel (1, 1'), dans lequel on peut imprimer au dispositif porte-outil (2) un mouvement simultané d'élévation et de rotation, un mécanisme de rotation (3) qui est réalisé pour imprimer le mouvement de rotation au dispositif porte-outil (2), et un mécanisme d'élévation (4) qui est réalisé pour imprimer le mouvement d'élévation au dispositif porte-outil (2), dans lequel le mécanisme de rotation (3) présente au moins un élément côté menant (3A) qui présente une roue dentée (3A') et un élément côté mené (3B) prévu sur le dispositif porte-outil (2), et dans lequel le mécanisme d'élévation (4) présente au moins un élément côté menant (4A) et un élément côté mené (4B) prévu sur le dispositif porte-outil (2), dans lequel l'élément côté menant (3A) du mécanisme de rotation (3) et l'élément côté menant (4A) du mécanisme d'élévation (4) sont disposés sur un unique arbre d'entraînement commun (5), dans lequel on peut imprimer à l'arbre d'entraînement (5) un mouvement de rotation autour d'un axe de rotation (6) et peut entraîner le mécanisme de rotation (3) et le mécanisme d'élévation (4) en commun et exclusivement grâce à cet unique arbre d'entraînement commun (5), dans lequel la roue dentée côté menant (3A') est disposée de façon concentrique autour de l'axe de rotation (6) de l'unique arbre d'entraînement commun (5), dans lequel
le mécanisme d'élévation (4) et le mécanisme de rotation (3) sont réalisés au moins selon l'une des caractéristiques (i)-(iii) suivantes, moyennant quoi on peut imprimer au dispositif porte-outil (2) un mouvement continu d'élévation et de rotation simultané:
(i) l'élément côté mené (3B) du mécanisme de rotation (3) est relié de manière fixe au dispositif porte-outil (2), moyennant quoi l'élément côté mené (3B) du mécanisme de rotation (3) peut être déplacé avec le dispositif porte-outil (2) de manière rotative et le long d'un axe médian ou de rotation (19) du dispositif porte-outil (2);
(ii) l'élément côté menant (3A) du mécanisme de rotation (3) et l'élément côté menant (4A) du mécanisme d'élévation (4) présentent respectivement une extrémité libre, dans lequel l'extrémité libre de l'élément côté menant (4A) du mécanisme d'élévation (4) fait au moins légèrement saillie par rapport à l'extrémité libre de l'élément côté menant (3A) du mécanisme de rotation (3);
(iii) l'élément côté mené (3B) du mécanisme de rotation (3) et l'élément côté mené (4B) du mécanisme d'élévation (4) sont espacés l'un de l'autre de telle sorte que respectivement seulement l'élément côté menant (3A) du mécanisme de rotation (3) entre en liaison de génération et/ou transmission de mouvement avec l'élément côté mené (3B) du mécanisme de rotation (3), et l'élément côté menant (4A) du mécanisme d'élévation (4) entre en liaison de génération et/ou transmission de mouvement avec l'élément côté mené (4B) du mécanisme d'élévation (4), et dans lequel
la roue dentée côté menant (3A') présente une gaine (7) s'étendant dans une partie de manche (12) de l'instrument manuel (1, 1') dans laquelle au moins une partie de l'unique arbre d'entraînement commun (5) est réceptionnée.

2. Instrument manuel médical, en particulier dentaire (1, 1') selon la revendication 1, **caractérisé en ce**
**que** l'élément côté mené (3B) du mécanisme de rotation (3) présente une roue dentée.

3. Instrument manuel médical, en particulier dentaire (1, 1') selon la revendication 1 ou 2, **caractérisé en ce**
**que** le mécanisme d'élévation (4) et le mécanisme de rotation (3) sont réalisés en tant que mécanismes séparés de sorte qu'ils génèrent et/ou transmettent le mouvement d'élévation et le mouvement de rotation de manière séparée l'un de l'autre, en particulier au moyen de pièces différentes.

4. Instrument manuel médical, en particulier dentaire (1, 1') selon la revendication 1, 2 ou 3, **caractérisé en ce**
**que** l'au moins une partie de l'unique arbre d'entraînement commun (5) est réceptionnée dans la gaine (7) par compression.

5. Instrument manuel médical, en particulier dentaire (1, 1') selon l'une des revendications précédentes, **caractérisé en ce**
**qu'**au moins une partie de l'élément côté menant (4A) du mécanisme d'élévation (4) s'étend à travers la roue dentée côté menant (3A'), de sorte que les dents de la roue dentée côté menant (3A') entourent l'élément côté menant (4A) du mécanisme d'élévation (4).

6. Instrument manuel médical, en particulier dentaire (1, 1') selon l'une des revendications précédentes, **caractérisé en ce**
**que** l'élément côté menant (4A) du mécanisme d'élévation (4) est disposé de manière concentrique autour d'un axe de rotation (6) de l'unique arbre d'entraînement commun (5).

7. Instrument manuel médical, en particulier dentaire (1, 1') selon la revendication 6, **caractérisé en ce**
**que** l'élément côté menant (4A) du mécanisme d'élévation (4) présente une goupille polygonale (4A') de préférence réalisée d'une seule pièce avec l'unique arbre d'entraînement commun (5), laquelle se met en prise avec l'élément côté mené (4B) du mécanisme d'élévation (4) au niveau du dispositif porte-outil (2).

8. Instrument manuel médical, en particulier dentaire (1, 1') selon la revendication 7, **caractérisé en ce**
**que** la goupille polygonale (4A') est réalisée à courbe de largeur constante.

9. Instrument manuel médical, en particulier dentaire (1, 1') selon la revendication 7 ou 8, **caractérisé en ce**
**que** la goupille polygonale (4A') présente des côtés extérieurs (8) bombés vers l'extérieur.

10. Instrument manuel médical, en particulier dentaire (1, 1') selon la revendication 9, **caractérisé en ce**
**qu'**en section transversale, le rayon des côtés extérieurs (8) bombés vers l'extérieur de la goupille polygonale (4A') est supérieur au rayon de l'enveloppe extérieure cylindrique (9) de l'unique arbre d'entraînement commun (5).

11. Instrument manuel médical, en particulier dentaire (1, 1') selon l'une des revendications 7 - 10, **caractérisé en ce**
**que** la goupille polygonale (4A') présente trois ou cinq sommets (10).

12. Instrument manuel médical, en particulier dentaire (1, 1') selon l'une des revendications 1-5, **caractérisé en ce**
**que** l'élément côté menant (4A) du mécanisme d'élévation (4) est disposé de manière excentrique par rapport à un axe de rotation (6) de l'unique arbre d'entraînement commun (5).

13. Instrument manuel médical, en particulier dentaire (1, 1') selon la revendication 12, **caractérisé en ce**
**que** l'élément côté menant (4A) du mécanisme d'élévation (4) présente une goupille cylindrique (4A") qui se met en prise avec l'élément côté mené (4B) du mécanisme d'élévation (4) au niveau du dispositif porte-outil (2).

14. Instrument manuel médical, en particulier dentaire (1, 1') selon l'une des revendications précédentes, **caractérisé en ce**
**que** l'élément côté menant (4A) du mécanisme d'élévation (4) est relié d'un seul tenant à l'unique arbre d'entraînement commun (5).

15. Instrument manuel médical, en particulier dentaire (1, 1') selon l'une des revendications précédentes, **caractérisé en ce**
**que** l'élément côté mené (4B) du mécanisme d'élévation (4) présente un retrait, lequel est fermé de manière circulaire ou de forme annulaire.
